(19) Europäisches Patentamt
European Patent Office
Office européen des brevets

(11) **EP 3 448 340 B1**

(12) **EUROPÄISCHE PATENTSCHRIFT**

(45) Veröffentlichungstag und Bekanntmachung des Hinweises auf die Patenterteilung:
**08.04.2020   Patentblatt 2020/15**

(21) Anmeldenummer: **17719486.7**

(22) Anmeldetag: **25.04.2017**

(51) Int Cl.:
*A61F 13/38* *(2006.01)*        *A61F 15/00* *(2006.01)*
*A61B 17/32* *(2006.01)*

(86) Internationale Anmeldenummer:
**PCT/EP2017/000524**

(87) Internationale Veröffentlichungsnummer:
**WO 2017/186349 (02.11.2017 Gazette 2017/44)**

(54) **APPLIKATIONSHILFE FÜR DIE BEHANDLUNG VON WUNDEN**

APPLICATION AID FOR THE TREATMENT OF WOUNDS

DISPOSITIF APPLICATEUR POUR LE TRAITEMENT DE PLAIES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **28.04.2016   DE 202016002788 U**

(43) Veröffentlichungstag der Anmeldung:
**06.03.2019   Patentblatt 2019/10**

(73) Patentinhaber: **Lohmann & Rauscher GmbH 2525 Schönau/Triesting (AT)**

(72) Erfinder:
 • **NÖLL, Corinna 25451 Quickborn (DE)**
 • **HARREITHER, Wolfgang 2514 Traiskirchen (AT)**
 • **TÜRTSCHER; Marko 6835 Muntlix (AT)**

(74) Vertreter: **Seranski, Klaus Boehmert & Boehmert Anwaltspartnerschaft mbB Pettenkoferstraße 22 80336 München (DE)**

(56) Entgegenhaltungen:
**DE-T2- 69 032 623     GB-A- 854 163**

• **Lohmann & Rauscher: "Debrisoft Lolly (Deutsch)", YouTube, 7. Januar 2016 (2016-01-07), Seiten 1-1, XP054977395, Gefunden im Internet: URL:https://www.youtube.com/watch?v=QrBdo k 1I6eE [gefunden am 2017-05-30]**
• **Anonymous: "Fluid Control Products", , 7. Februar 2016 (2016-02-07), XP055351551, Gefunden im Internet: URL:http://web.archive.org/web/20160207175 247/http://www.beaver-visitec.com/upload/F luid-Control-Sell-Sheet.pdf [gefunden am 2017-03-03]**
• **Anonymous: "Innovative Debridement Device for Deep and Hard-To-Reach Wounds", , 14. April 2016 (2016-04-14), XP055376812, Gefunden im Internet: URL:http://www.lohmann-rauscher.us/uploads /media/Press_Release_Lohmann_Rauscher_De br isoft_Lolly.pdf [gefunden am 2017-05-30]**
• **Activa Healthcare: "Debrisoft Lolly", YouTube, 28. Juni 2016 (2016-06-28), Seiten 1-1, XP054977394, Gefunden im Internet: URL:https://www.youtube.com/watch?v=jX_aa W zk6Zc [gefunden am 2017-05-30]**
• **CATHERINE MEADS ET AL: "The Debrisoft Monofilament Debridement Pad for Use in Acute or Chronic Wounds: A NICE Medical Technology Guidance", APPLIED HEALTH ECONOMICS AND HEALTH POLICY, Bd. 13, Nr. 6, 1. Dezember 2015 (2015-12-01), Seiten 583-594, XP055377171, ISSN: 1175-5652, DOI: 10.1007/s40258-015-0195-0**

EP 3 448 340 B1

## Beschreibung

Die Erfindung betrifft eine Applikationshilfe für die Behandlung von Wunden. Die Applikationshilfe soll insbesondere zum Debridement und/oder zur Reinigung von Wunden einsetzbar sein.

Als Debridement wird allgemein die Entfernung von Verunreinigungen wie etwa abgestorbenem Gewebe, Debris und Exsudat sowie Schuppen und Keratosen aus Wunden und der Wundumgebungshaut bezeichnet. Ziel ist eine Reinigung der Wunde, eine verbesserte Gewährleistung der Wundbeobachtung sowie eine Förderung der Heilung. Das Debridement insbesondere von tiefen - auch chirurgisch invasiven - Wunden wie etwa diabetischen Ulcera, arteriellen und venösen Ulcera, Dekubitalulcera, postoperativen und sekundärheilenden Wunden, Traumata, Verbrennungen und Verbrühungen ist typischerweise zur Beschleunigung des Heilprozesses und zum Verhindern einer sekundären Infektion der Wunde erforderlich.

Hierzu können Applikationshilfen mit einem Applikationsstab verwendet werden, an dessen vorderem Ende ein Wundbehandlungskopf angebracht ist. Der Applikationsstab kann eine Länge haben, die eine Behandlung und Reinigung auch tiefer Wunden mittels des Wundbehandlungskopfs ermöglicht.

Herkömmliche Applikationshilfen haben allerdings mitunter zu Verletzungen der Wunde oder der Wundumgebung geführt.

In Anbetracht dieser Probleme ist es die Aufgabe der vorliegenden Erfindung, eine Applikationshilfe zur Behandlung von akuten und chronischen, oberflächlichen bis tiefen Wunden bereitzustellen, die eine gute Reinigungsleistung ohne die Gefahr von Verletzungen ermöglicht.

Diese Aufgabe wird erfindungsgemäß durch die im unabhängigen Patentanspruch 1 angegebene Weiterbildung der eingangs beschriebenen Applikationshilfe gelöst.

Die Erfindung geht auf die Erkenntnis zurück, dass ein starrer Applikationsstab insbesondere beim Einsatz in tiefen Wunden zu Verletzungen führen kann, wenn der Anwender die Wunde unter Anwendung von zu hoher Druckkraft behandelt. Bspw. kann ein steifer bzw. unnachgiebiger Applikationsstab zu Druckstellen oder Rissen in der Wundumgebung führen, wenn er in eine tiefe Wunde eingeführt oder darin bewegt wird.

Demgegenüber gibt der biegsame Applikationsstab der erfindungsgemäßen Applikationshilfe bei zu hoher Kraftausübung durch den Anwender nach, so dass eine Verletzung der Wunde und der Wundumgebung verhindert werden kann.

Vorzugsweise verformt sich der Applikationsstab der erfindungsgemäßen Applikationshilfe bei Ausübung einer Biegekraft in einer Querrichtung des Stabs bis hin zu einer Elastizitätsgrenze flexibel bzw. elastisch. Mit anderen Worten verbiegt sich der Applikationsstab bei Einwirkung einer Biegekraft senkrecht zu der Längsrichtung des Applikationsstabs in reversibler Weise und kehrt bei Aufhebung der Krafteinwirkung in seinen Ursprungszustand zurück. Mit der erfindungsgemäßen Applikationshilfe mit dem biegsamen, insbesondere dem elastisch verformbaren Applikationsstab ist ein Debridement von oberflächlichen bis tiefen Wunden ohne die Gefahr einer Verletzung der Wunde möglich.

Die Handhabbarkeit der erfindungsgemäßen Applikationshilfe kann dadurch verbessert werden, dass ein hinterer Abschnitt des Applikationsstabs als ein Haltegriffabschnitt ausgebildet ist, der bspw. die Form eines Handgriffs haben kann. Bei einigen Ausführungsformen ist der Haltegriffabschnitt im Hinblick auf eine verbesserte Griffigkeit gekrümmt und/oder profiliert. Bspw. weist der Haltegriffabschnitt sich in der Längsrichtung des Applikationsstabs erstreckende Wellungen zum Einlegen von Fingern auf. Ein Verrutschen des Applikationsstabs relativ zu der Hand des Anwenders kann zuverlässig verhindert werden.

Ein vorderer Abschnitt des Applikationsstabs kann als ein Befestigungsabschnitt zum Befestigen eines Wundreinigungskopfs ausgebildet sein, wobei der Wundreinigungskopf bspw. ein Wundreinigungstuch oder ein Wundreinigungspad sein kann. Nach der Befestigung des Wundreinigungskopfs an dem Befestigungsabschnitt kann der Wundreinigungskopf den Befestigungsabschnitt vorzugsweise vollständig umlaufen bzw. umhüllen. Hierdurch wird sichergestellt, dass ausschließlich der Wundreinigungskopf, nicht jedoch das vordere Ende des Applikationsstabs in Kontakt mit dem Wundgewebe kommt. Als Wundreinigungstuch oder Wundreinigungspad wird vorliegend ein flächiges textiles Gebilde, einschließlich Watte, Vlies, Gewebe etc. bezeichnet, das je nach Bedarf und je nach Einsatzbereich aus natürlichen und/oder synthetischen Fasern besteht und die gewünschten Reinigungseigenschaften aufweist.

Die Flexibilität des Applikationsstabs wird erfindungsgemäß dadurch verbessert werden, dass zwischen dem Haltegriffabschnitt und dem Befestigungsabschnitt ein Biegeabschnitt mit erhöhter Biegsamkeit angeordnet ist.

Vorzugsweise weist der Applikationsstab in dem Biegeabschnitt einen gegenüber dem Haltegriffabschnitt verkleinerten Stabquerschnitt und/oder zumindest eine sich quer zur Längsrichtung des Applikationsstabs erstreckende Kerbe oder Vertiefung zum Erhöhen der Biegsamkeit auf.

Im Hinblick auf eine gute Reinigungsleistung bei Anwendung in tiefen Wunden hat es sich als zweckmäßig herausgestellt, dass sich der Haltegriffabschnitt über mehr als 1/3 und weniger 2/3 der Länge des Applikationsstabs erstreckt, und/oder sich der Befestigungsabschnitt über mehr als 1/5 und weniger als 1/3 der Länge des Applikationsstabs erstreckt. Bei einer Gesamtlänge des Applikationsstabs zwischen 14 cm und 18 cm kann sich der Haltegriffabschnitt bspw. über eine Länge zwischen 6 cm und 10 cm erstrecken und/oder der Befestigungsabschnitt über eine Länge zwischen 4 cm und 6 cm, insbesondere über etwa 5 cm, erstrecken. Der Befestigungsabschnitt ist vorzugsweise vollständig von dem Wundreinigungskopf bedeckt bzw. umhüllt.

Nicht nur eine zu hohe Steifheit des Applikationsstabs, sondern auch eine zu hohe Flexibilität kann sich bei der Wundbehandlung als nachteilig erweisen. Bspw. führt eine zu hohe Biegsamkeit des Applikationsstabs dazu, dass der Anwender das Gefühl für die Kraft der Anwendung verliert. Der Anwender glaubt zum Beispiel, die zum Erhalt einer erwünschten Reinigungsleistung erforderliche Kraft bereits aufzuwenden, während tatsächlich aufgrund einer hohen Durchbiegung des Applikationsstabs der nötige Druck für die Anwendung nicht erreicht wird.

Eine besonders gute Reinigungsleistung kann mit einem Applikationsstab erzielt werden, der eine Biegedehnung von 5% bei Einwirkung einer Biegekraft senkrecht zu seiner Längsrichtung erfährt, die einen Wert von 60 N oder weniger, vorzugsweise 45 N oder weniger, besonders bevorzugt 39 N oder weniger und/oder 15 N oder mehr, vorzugsweise 21 N oder mehr, besonders bevorzugt 27 N oder mehr aufweist. Bei einer besonders bevorzugten Ausführungsform der Erfindung führt die Einwirkung einer Biegekraft von 33 N (+/-6 N) auf den Applikationsstab zu einer Biegedehnung von 5% (bei einer Temperatur von 20+/-2°C).

Vorzugsweise wirkt die Biegekraft dabei auf den Biegeabschnitt des Applikationsstabs, während der vordere und der hintere Abschnitt des Applikationsstabs jeweils auf einer Auflagefläche einer 3-Punkt-Biegevorrichtung mit einer Stützweite St zwischen St=40 mm und St=60 mm aufliegen.

Im Hinblick auf die Definition der "Biegedehnung" wird auf die Norm EN ISO 178 (Juni 2003) verwiesen, deren Inhalt durch Verweis vollständig in die vorliegende Offenbarung aufgenommen wird.

Erfindungsgemäß ist der Applikationsstab derart eingerichtet, dass er sich in einer 3-Punkt-Biegevorrichtung mit einer Stützweite zwischen St = 40 mm und St = 60 mm (insbesondere St = 48 mm) bei Einwirkung einer Biegekraft zwischen 21 N und 45 N, insbesondere von etwa 33 N, auf den Biegeabschnitt senkrecht zu der Längsrichtung des Applikationsstabs um etwa s = 5 mm bis s = 8 mm (insbesondere um etwa s = 6,4 mm) durchbiegt (bei einer Temperatur von 20+/-2°C).

Bei einer Stabdicke (Dicke des Stabs in Richtung der Biegekraft) von h = 3 mm im Bereich des Biegeabschnitts ergibt sich aus der Formel (1) der Norm EN ISO 178 bei einer Durchbiegung von s = 6,4 mm eine Biegedehnung von 5% wie folgt:

$$\varepsilon_f = 600 * s * h / St^2 \% \qquad (1)$$

Im Hinblick auf eine Erhöhung der Anwendungssicherheit hat es sich als zweckmäßig herausgestellt, dass der Applikationsstab bruchsicher ausgebildet ist. Mit anderen Worten ist der Applikationsstab besonders weit quer zu seiner Längsrichtung vorzugsweise elastisch auslenkbar, bevor er bricht oder knickt.

Als ausreichend bruchsicher hat sich ein Applikationsstab herausgestellt, der eine Bruchdehnung von 6% oder mehr, vorzugsweise 8% oder mehr, insbesondere etwa 16% und/oder 50% oder weniger aufweist.

Im Rahmen dieser Beschreibung und der Ansprüche bezeichnet der Ausdruck "Bruchdehnung" die Biegedehnung - definiert gemäß der Norm EN ISO 178 - bei der der Applikationstab zum ersten Mal bricht. Die Einhaltung einer Obergrenze der Bruchdehnung von 50% kann hilfreich sein, um die Ausübung einer übermäßigen Kraft auf die Wunde zu vermeiden.

Vorzugsweise wirkt die Biegekraft dabei auf den Biegeabschnitt des Applikationsstabs, während der vordere und der hintere Stababschnitt jeweils auf einer Auflagefläche einer 3-Punkt-Biegevorrichtung mit einer Stützweite St zwischen St = 40 mm und St = 60 mm aufliegen.

Alternativ oder zusätzlich ist der Applikationsstab derart eingerichtet, dass er in einer 3-Punkt-Biegevorrichtung mit einer Stützweite zwischen St = 40 mm und St = 60 mm (insbesondere St = 48 mm) bei Einwirkung einer Biegekraft auf den Biegeabschnitt senkrecht zu der Längsrichtung des Applikationsstabs selbst dann noch nicht bricht oder knickt, wenn sich eine Durchbiegung s des Applikationsstabs senkrecht zu seiner Längsrichtung von s = 20 mm ergibt (bei einer Temperatur von 20+/-2°C).

Gemäß der vorliegende Erfindung umfasst die Applikationshilfe zusätzlich einem an einem vorderen Abschnitt des Applikationsstabs befestigten Wundreinigungskopf, insbesondere in Form eines Wundreinigungspads oder Wundreinigungstuchs. Unter einem Wundreinigungstuch wird ein für die Reinigung einer Wunde geeignetes Textil (natürlich und/oder synthetisch) verstanden.

Eine gute Reinigungswirkung ergibt sich dadurch, dass der Wundreinigungskopf ein Faserpad, insbesondere ein Faserpad mit synthetischen Fasern ist, die vorzugsweise eine Faserstärke zwischen 0,5 und 20 dtex aufweisen. Bei bevorzugten Ausführungsformen der Erfindung beträgt die Faserstärke zwischen 3 und 12 dtex, insbesondere zwischen 5 und 8 dtex. Bei einer besonders bevorzugten Ausführungsform der Erfindung liegt eine Mischung von Fasern mit einer Stärke von etwa 3,3 dtex und etwa 6,7 dtex in einem Mischungsverhältnis von etwa 50:50 vor. (1 dtex = g/10.000 m).

Dabei kann das Faserpad zumindest eine Trägerschicht und an der Trägerschicht angeordnete und von der Trägerschicht abstehende Fäden aus synthetischen Fasern, insbesondere Kunststofffasern aufweisen, die vorzugsweise ein Flor mit einer Florhöhe zwischen 3 und 30 mm bilden. Bei bevorzugten Ausführungsformen der Erfindung liegt die Florhöhe zwischen 3 und 13 mm, sie beträgt besonders bevorzugt 8 bis 12 mm. Das Flor kann aus Fasern unterschiedlicher Faserstärken gebildet sein. Es können zur Bildung des Flors auch Fasern aus unterschiedlichen Materialien eingesetzt werden. In allen Fällen ist es bevorzugt, wenn die Faserstärke sämtlicher Fasern zwischen 0,5 und 20 dtex beträgt.

Die Reinigungswirkung kann dadurch weiter verbessert werden, dass die Fäden schräg zu ihrer Längserstreckung verlaufende und vorzugsweise abgeschnittene Enden bzw. Endflächen aufweisen.

Im Hinblick auf den Aufbau und die Merkmale des einsetzbaren Wundreinigungskopfs wird auf die Druckschrift WO 2010/085831 A1 verwiesen. Der an dem vorderen Ende des Applikationsstabs der erfindungsgemäßen Applikationshilfe befestigbare oder befestigte Wundreinigungskopf kann einige oder alle Merkmale der in dieser Druckschrift beschriebenen Wundreinigungstücher einzeln oder in beliebiger Kombination aufweisen.

Eine Verletzung der Wunde durch den Applikationsstab kann dadurch zuverlässig verhindert werden, dass der Wundreinigungskopf den vorderen Abschnitt des Applikationsstabs vollständig umläuft oder umhüllt. Insbesondere umläuft der Wundreinigungskopf den zur Befestigung des Wundreinigungskopfs ausgebildeten Befestigungsabschnitt des Applikationsstabs teilweise oder vollständig.

In einigen Ausführungsformen kann der Wundreinigungskopf derart an dem Befestigungsabschnitt des Applikationsstabs angebracht sein, dass er den Befestigungsabschnitt vollständig umhüllt, wobei der Applikationsstab in Richtung auf den hinteren Abschnitt aus einer in dem Wundreinigungskopf gebildeten Durchgriffsöffnung an dessen hinterem Ende austreten kann.

Alternativ oder zusätzlich umläuft der Wundreinigungskopf den vorderen Abschnitt des Applikationsstabs vollständig, wobei eine erste Lage des als Wundreinigungstuch ausgebildeten Wundreinigungskopfs auf einer ersten Seite des Applikationstabs (bspw. oberhalb) und eine zweite Lage des Wundreinigungskopfs auf der entgegengesetzten Seite des Applikationsstabs (bspw. unterhalb) angeordnet ist, so dass der vordere Abschnitt des Applikationsstabs zwischen den zwei Lagen angeordnet ist. Der Wundreinigungskopf kann bspw. ein einmal oder mehrmals um den vorderen Abschnitt des Applikationsstabs gewickeltes Pad oder Tuch aufweisen.

Ein Ablösen des Wundreinigungskopfs von dem Applikationsstab bei der Wundbehandlung kann durch eine hohe Verbindungsfestigkeit zwischen dem Applikationsstab und dem Wundbehandlungskopf verhindert werden. Vorzugsweise ist die Verbindungsfestigkeit zwischen dem Wundbehandlungskopf und dem Applikationsstab bei einem Abziehen des Wundreinigungskopfs von dem Applikationsstab in Längsrichtung des Applikationsstabs größer als 25 N, besonders bevorzugt größer als 50 N. Insbesondere ist die Verbindungsfestigkeit 70 N oder größer. Unter einer Verbindungsfestigkeit von 70 N oder mehr wird erfindungsgemäß verstanden, dass sich der Wundreinigungskopf nicht von dem Applikationsstab ablöst, wenn daran mit einer Abziehkraft von 70 N oder mehr in Längsrichtung des Applikationsstabs gezogen wird.

Bei einer besonders bevorzugten Ausführungsform der Erfindung weist der Wundreinigungskopf eine von dem Applikationsstab durchlaufene Durchgriffsöffnung auf, durch die der Applikationsstab in Richtung auf den hinteren Abschnitt aus dem Wundreinigungskopf austreten kann. Der von dem Wundreinigungskopf umlaufene vordere Abschnitt des Applikationsstabs kann von dem Applikationsstab abstehende Vorsprünge wie etwa Widerhaken aufweisen, die ein Hindurchgleiten des Applikationsstabs durch die Durchgriffsöffnung verhindern. Auf diese Weise kann die Verbindungsfestigkeit zwischen dem Applikationsstab und dem Wundreinigungskopf weiter verbessert werden.

Alternativ oder zusätzlich kann der Wundreinigungskopf mittels einer oder mehrerer Befestigungsnahten an dem Applikationsstab befestigt sein. Beispielsweise weist der vordere Rand des Wundreinigungskopfs und/oder der dem vorderen Rand entgegengesetzte hintere Rand des Wundreinigungskopfs, der dem Haltegriffabschnitt des Applikationsstabs zugewandt ist, zumindest eine Befestigungsnaht auf.

Die Verbindungsfestigkeit kann weiter verbessert werden, indem der dem Haltegriffabschnitt zugewandte hintere Rand des Wundreinigungskopfs beidseitig des Applikationsstabs eine Befestigungsnaht aufweist. Beispielsweise kann der dem Haltegriffabschnitt zugewandte hintere Rand des Wundreinigungskopfs eine Stufe oder einen Vorsprung des Applikationsstabs übergreifend vernäht sein. Auf diese Weise kann ein Abziehen des Wundreinigungskopfs von dem Applikationsstab zuverlässig verhindert werden. Insbesondere kann verhindert werden, dass der Applikationsstab bei der Wundbehandlung versehentlich durch eine Durchgriffsöffnung des Wundreinigungskopfs gleiten kann.

Die Sicherheit bei der Anwendung im chirurgisch-invasiven Bereich kann dadurch verbessert werden, dass zumindest eine der Befestigungsnahten einen Röntgenkontrastfaden (RK-Faden), insbesondere einen mit Bariumsulfat beladenen Kunststofffaden aufweist. Ein RK Faden kann bspw. mit Bariumsulfat beladenes Polypropylen aufweisen.

Alternativ oder zusätzlich kann die erfindungsgemäße Applikationshilfe darin eingearbeitete oder daran angeordnete RK-Chips, RK-Blättchen und/oder andere Röntgenkontrastmittel aufweisen.

Im Hinblick auf eine weitere Erhöhung der Verbindungsfestigkeit zwischen dem Applikationsstab und dem Wundbehandlungskopf hat es sich als zweckmäßig erwiesen, dass der Applikationsstab eine Öffnung oder eine Aussparung aufweist, durch die der Wundbehandlungskopf zusätzlich am Applikationsstab gesichert werden kann. Bspw. ist eine auf einer ersten Seite des Applikationsstabs angeordnete Lage des Wundreinigungskopfs durch die Öffnung hindurch mit einer auf einer zweiten Seite des Applikationsstabs angeordneten Lage des Wundreinigungskopfs verbunden, bspw. vernäht.

Der Applikationsstab kann diese Öffnung in seinem vorderen Abschnitt aufweisen, wobei die Öffnung bspw. als ein sich in Längsrichtung des Applikationsstabs erstreckender Schlitz ausgebildet sein kann. Eine Schlitzöffnung eignet sich besonders gut zum Durchnähen einer Sicherungsnaht durch den Applikationsstab. Die Schlitzöffnung kann eine Länge von 1 cm oder mehr oder 2 cm oder mehr und/oder 5 cm oder weniger aufweisen.

Alternativ oder zusätzlich kann der Wundreinigungskopf mittels Ultraschallschweißen an dem Applikationsstab befestigt sein.

Alternativ oder zusätzlich kann der Wundreinigungskopf über einen oder mehrere Widerhaken an dem Applikationsstab befestigt sein. Bspw. weist der Applikationsstab in seinem Befestigungsabschnitt einen oder mehrere von dem Applikationsstab abstehende Widerhaken auf, die den Wundreinigungskopf am Applikationsstab sichern.

Alternativ oder zusätzlich ist der Wundreinigungskopf mittels eines Haftmittels an dem Applikationsstab befestigt. Bspw. kann der Wundreinigungskopf an den Applikationsstab angeklebt sein. Durch ein Haftmittel kann eine besonders hohe Verbindungsfestigkeit bereitgestellt werden.

Alternativ oder zusätzlich kann der Wundreinigungskopf mittels eines vorzugsweise lösbaren Klicksystems an dem Applikationsstab befestigt sein. Hierdurch kann eine Wiederverwendung des Stabs und/oder Pads ermöglicht werden.

Alternativ oder zusätzlich kann der Wundreinigungskopf mittels eines Klettverschlusses an dem Applikationsstab befestigt sein. Hierdurch kann eine Wiederverwendung des Stabs und/oder Pads ermöglicht werden.

Alternativ oder zusätzlich kann der Wundreinigungskopf mittels eines Formverschlusses an dem Applikationsstab befestigt sein.

Der Wundreinigungskopf kann an den vorgesehenen Einsatzbereich angepasste Abmessungen haben. Ein kleiner Wundreinigungskopf kann bspw. leichter für Wundtaschen verwendet werden.

Bei einer bevorzugten Ausführungsform der Erfindung beträgt die Länge des Wundreinigungskopfs in Längsrichtung des Applikationsstabs 3 cm oder mehr und 10 cm oder weniger, insbesondere etwa 5 cm.

Dabei kann die Breite des Wundreinigungskopfs in einer senkrecht zur Längsrichtung des Applikationsstabs verlaufenden Breitenrichtung 1 cm oder mehr und 5 cm oder weniger, insbesondere etwa 2 cm betragen.

Das Erkennen der Wundtiefe durch den Anwender kann dadurch ermöglicht werden, dass der Applikationsstab eine Längenskala aufweist. Die Längenskala kann die Entfernung einer oder mehrerer Positionen am Applikationsstab von dem vorderen Ende des Applikationsstabs angeben.

Alternativ oder zusätzlich können andere Beschriftungen, Logos, Stichcodes; QR-Codes, RFID-Chips o.dgl. an dem Applikationsstab angeordnet sein, um dem Anwender weitere Informationen zur Verfügung zu stellen.

Bei einer besonders bevorzugten Ausführungsform der Erfindung ist ein Haltegriffabschnitt des Applikationsstabs rutschfest ausgebildet, so dass er auch mit Handschuhen ergriffen werden kann, ohne dass die Gefahr des Abrutschens besteht.

Im Hinblick auf eine verbesserte Griffigkeit des Haltegriffabschnitts hat es sich als zweckmäßig erwiesen, dass der Haltegriffabschnitt eine Profilierung aufweist, insbesondere eine Wellung, ein sich in Längsrichtung des Applikationsstabs in Richtung auf dessen hinteres Ende zumindest abschnittsweise erweiterndes Querschnittprofil und/oder eine ergonomische Form zum Erhöhen der Rutschfestigkeit.

Die Handhabbarkeit der erfindungsgemäßen Applikationshilfe kann dadurch weiter verbessert werden, dass der Applikationsstab zumindest abschnittsweise gebogen oder gekrümmt ist. Bspw. erstrecken sich ein hinterer Abschnitt des Applikationsstabs im Wesentlichen in einer ersten Ebene und ein vorderer Abschnitt des Applikationsstabs im Wesentlichen in einer bzgl. der ersten Ebene versetzten zweiten Ebene, wobei der mittlere Abschnitt des Applikationsstabs von der ersten Ebene in die zweite Ebene gekrümmt verläuft. Hierdurch kann sich ein Verlauf des Applikationsstabs nach Art einer Kelle ergeben, der bspw. im Hinblick auf die Anwendung bei tiefen Wunden bevorzugt sein kann.

Die Herstellung des Applikationsstabs kann dadurch vereinfacht werden, dass dieser zumindest teilweise und vorzugsweise vollständig aus Kunststoff, bspw. auf Polypropylen, gebildet ist. Bspw. kann der Applikationsstab einstückig aus Kunststoff geformt werden.

Im Hinblick auf die Behandlung tiefer Wunden hat sich eine Gesamtlänge des Applikationsstabs von 10 cm oder mehr und 20 cm oder weniger, insbesondere von etwa 15-16 cm als geeignet erwiesen.

Bei einer erfindungsgemäßen Applikationshilfe kann ein Bereich des Biegeabschnitts einen Werkstoff geringerer Biegefestigkeit aufweisen als die an den Biegeabschnitt angrenzenden Abschnitte.

Die Applikationshilfe kann im Hinblick auf die Anwendung in Wunden steril bzw. entkeimt sein.

In der folgenden Beschreibung wird die Erfindung unter Bezugnahme auf die beigefügten Zeichnungen erläutert. In der Zeichnung zeigt

Fig. 1      eine schematische Seitenansicht einer Applikationshilfe mit Wundbehandlungskopf gemäß der vorliegenden Erfindung;

Fig. 2      eine erste schematische Seitenansicht eines Applikationsstabs einer erfindungsgemäßen Applikationshilfe;

Fig. 3      eine um 90° gedrehte zweite schematische Seitenansicht des in Fig. 2 gezeigten Applikationsstabs;

Fig. 4a-4c      verschiedene Ansichten einer Applikationshilfe gemäß der vorliegenden Erfindung;

Fig. 5a-5c      verschiedene Ansichten einer Applikationshilfe gemäß der vorliegenden Erfindung;

Fig. 6a-6c      Seitenansichten verschiedener Applikationsstäbe von Applikationshilfen gemäß der vorliegenden Erfindung; und

Fig. 7      ein Verfahren zum Messen der Biegsamkeit von Applikationsstäben.

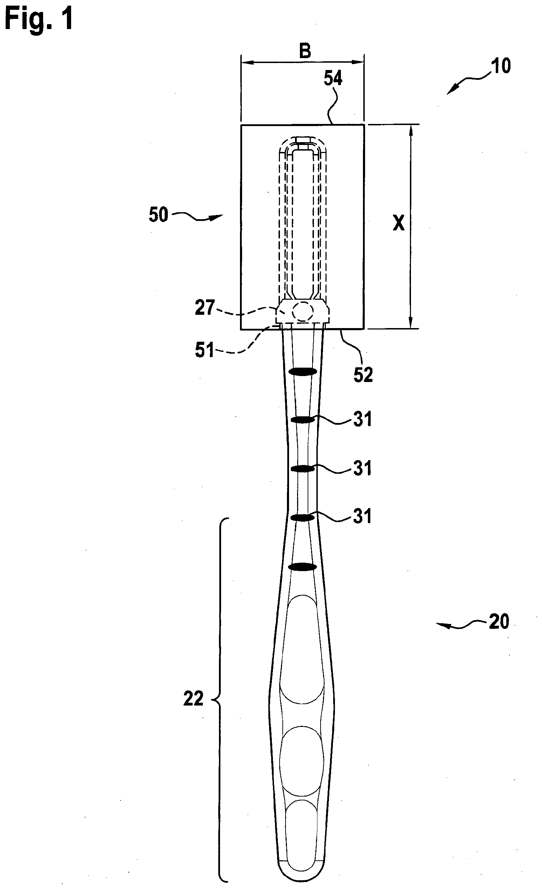

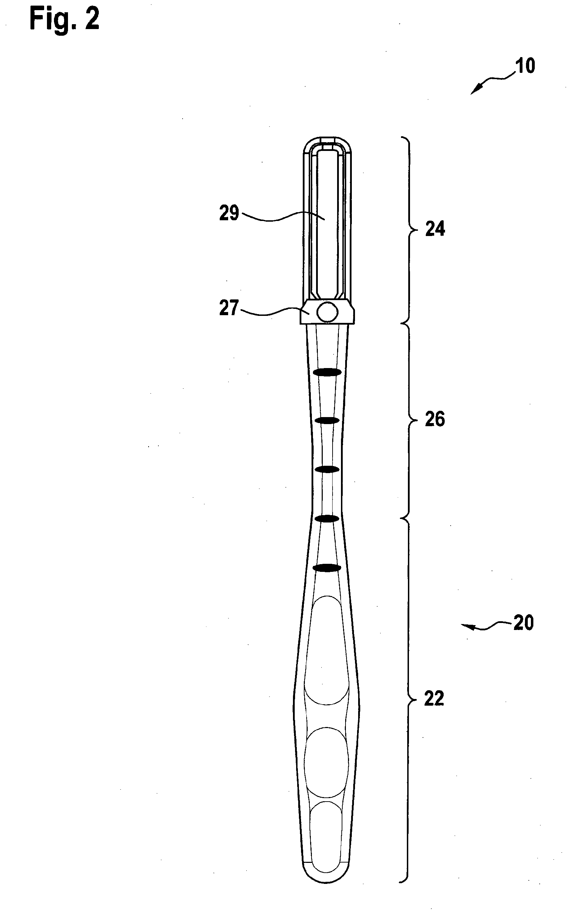

In Fig. 1 ist eine erfindungsgemäße Applikationshilfe 10 in einer schematischen Seitenansicht dargestellt. Die Applikationshilfe 10 besteht aus einem biegbaren Applikationsstab 20 und einem als Wundreinigungspad ausgebildeten Wundreinigungskopf 50, der an einem vorderen Abschnitt des Applikationsstabs 20 befestigt ist. Fig. 2 zeigt den Applikationsstab 20 ohne daran befestigten Wundreinigungskopf 50.

Ein hinterer Abschnitt des Applikationsstabs 20 ist als ein Haltegriffabschnitt 22 ausgebildet, ein mittlerer Abschnitt des Applikationsstabs 20 ist als eine Biegeabschnitt 26 mit erhöhter Biegsamkeit ausgebildet, und der vordere Abschnitt des Applikationsstabs ist als ein Befestigungsabschnitt 24 zum Befestigen des Wundreinigungskopfs 50 ausgebildet.

Bei der in Fig. 1 dargestellten Ausführungsform umläuft der nur schematisch angedeutete Wundreinigungskopf 50 den Befestigungsabschnitt 24 vollständig, so dass bei der Behandlung von Wunden mit der Applikationshilfe 10 ein direkter Kontakt zwischen der Wunde und dem vorderen Ende des Applikationsstabs 20 vermieden wird.

Der Applikationsstab 20 erstreckt sich in einer Längsrichtung und hat vorzugsweise eine Gesamtlänge L zwischen 10 cm und 20 cm, insbesondere etwa 15-16 cm. Der Haltegriffabschnitt 22 kann sich etwa über die Hälfte der Gesamtlänge des Applikationsstabs erstrecken, während der Befestigungsabschnitt an die Länge X des Wundreinigungskopfs angepasst sein kann und bspw. eine Länge von etwa 5 cm aufweisen kann. Bei einer bevorzugten Ausführungsform hat der Wundreinigungskopf eine Länge X von etwa 5 cm und eine Breite B von etwa 2-3 cm.

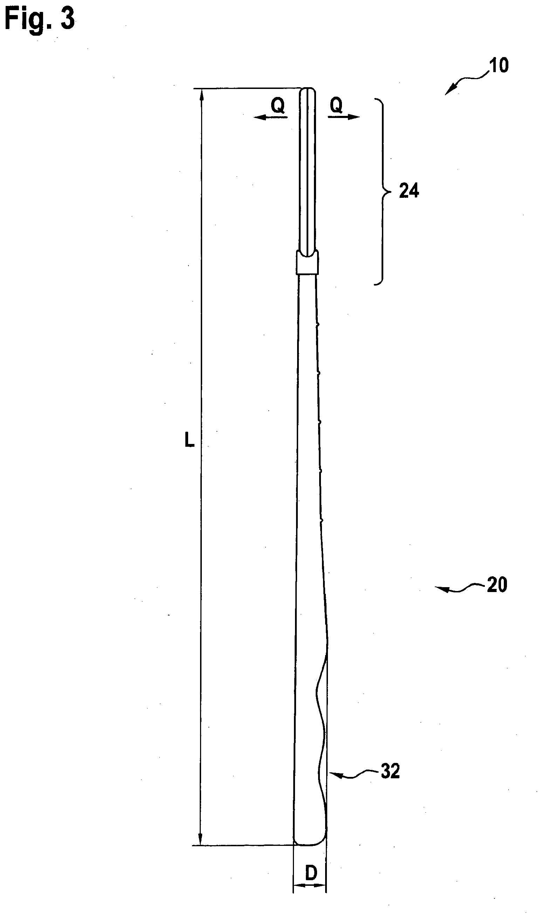

Der Applikationsstab 20 kann in dem Haltegriffabschnitt 22 eine Profilierung 32 wie etwa eine Wellung zum Verbessern seiner Griffigkeit aufweisen, und er kann ferner einen sich in Richtung auf den Wundreinigungskopf 50 verkleinernden Querschnitt aufweisen, wie bspw. in Fig. 3 angedeutet ist.

Fig. 3 zeigt einen Applikationsstab 20 einer erfindungsgemäßen Applikationshilfe 10 in einer um 90° gedrehten Seitenansicht. Die maximale Dicke D des Applikationsstabs in dem Haltegriffabschnitt 22 kann etwa 5-7 mm betragen, während der Biegeabschnitt 26 eine Dicke von nur etwa 2-4 mm, insbesondere etwa 3 mm haben kann.

In den Figuren 1 und 2 ist ferner eine in dem Biegeabschnitt 26 und in einem vorderen Bereich des Haltegriffabschnitts 22 angeordnete Skala 31 zum Ablesen einer Wundtiefe dargestellt.

Der Applikationsstab 20 ist elastisch verformbar und kehrt bei Auslenkung in einer Querrichtung Q wieder in seinen unbelasteten Zustand zurück.

Die Biegesteifigkeit des Applikationsstabs 20 ist dabei derart eingerichtet, dass sich der Applikationsstab bei einer übermäßigen Druckausübung durch den Anwender durchbiegt, wodurch Verletzungen der zu behandelnden Wunde verhindert werden können. Gleichzeitig ist die Biegesteifigkeit ausreichend hoch, so dass der Anwender nicht das Gefühl für die Kraft seiner Anwendung verliert.

Die Biegsamkeit der Applikationsstabs ist insbesondere derart eingerichtet, dass sich bei Einwirkung einer Biegekraft auf den Applikationsstab senkrecht zu dessen Längsrichtung mit einem zwischen 21 N und 45 N liegenden Wert, insbesondere von etwa 33 N, eine Biegedehnung des Applikationsstabs von etwa 5% ergibt.

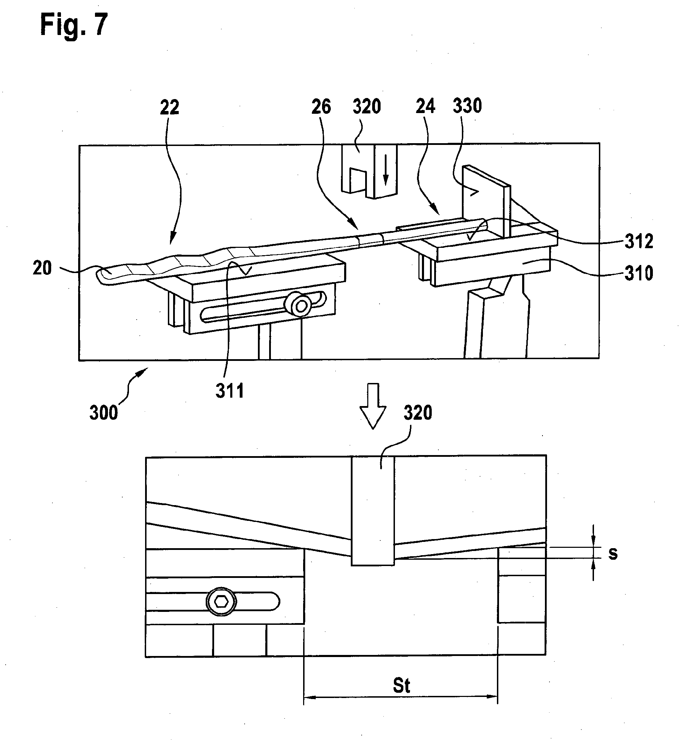

Unter Bezugnahme auf Fig. 7 wird im Folgenden ein Verfahren zum Bestimmen der Biegedehnung eines Applikationsstabs mittels einer 3-Punkt-Biegevorrichtung erläutert, wobei die Messung in Anlehnung an das in der Norm DIN EN ISO 178 beschriebene Verfahren durchgeführt wird.

Fig. 7 zeigt im oberen Teil ein Prüfgerät 300 mit einem Auflager 310 und einer darüber angeordneten Druckfinne 320. Das Auflager 310 weist eine erste Auflagefläche 311 zur Auflage des Haltegriffabschnitts 22 eines Applikationsstabs und eine davon beabstandete zweite Auflagefläche 312 zur Auflage des vorderen Abschnitts 24 des Applikationsstabs 20 auf. Der Biegeabschnitt 26 des Applikationstabs ist oberhalb eines Zwischenraums zwischen den beiden Auflageflächen 311, 312 angeordnet, wobei der Zwischenraum eine Stützweite St von St=40 mm oder mehr und St=60 mm oder weniger, insbesondere etwa St=48 mm hat. Eine vertikale Anschlagfläche 330 vereinfacht die korrekte Positionierung des Applikationsstabs auf dem Auflager 310.

Die Druckfinne 320 weist an ihrem unteren Ende eine Druckfläche auf und ist senkrecht zu der Längsrichtung des Applikationsstabs beweglich eingerichtet. Wie in dem unteren Teil von Fig. 7 dargestellt ist, wird die Druckfinne 320 unter Ausübung einer Biegekraft auf den Applikationsstab 20 von oben in den Zwischenraum zwischen den beiden Auflageflächen hineinbewegt, bis hin zu einer in Fig. 7 gezeigten voreinstellbaren Endstellung, in der sich eine vorgegebene Durchbiegung s des Applikationsstabs senkrecht zu dessen Längsrichtung ergibt. Die von der Druckfinne 320 in der Endstellung auf den Applikationsstab ausgeübte Biegekraft ist messbar.

Bei einer Stabdicke von h = 3 mm im Biegeabschnitt, auf den die Biegekraft einwirkt, ergibt sich aus der Formel ($\varepsilon_f = 600 * s * h / St^2$ %) bei einer Durchbiegung von etwa s=6,4 mm eine Biegedehnung von 5%.

Für eine solche Biegedehnung sollte vorzugsweise eine Biegekraft zwischen 21 N und 45 N erforderlich sein, insbesondere eine Biegekraft von etwa 33 N. Ein Applikationsstab erfüllt also insbesondere dann die bevorzugten Biegeeigenschaften, wenn sich bei einer von der Druckfinne ausgeübten Biegekraft von etwa 33 N eine Durchbiegung s zwischen 5 mm und 8 mm, insbesondere etwa 6,4 mm ergibt.

Ferner ist der erfindungsgemäße Applikationsstab vorzugsweise besonders bruchfest gegenüber einer Biegebelastung eingerichtet. Insbesondere sollte der Applikationsstab bei einer Biegedehnung von 8%, insbesondere selbst bei einer Biegedehnung von 16% noch nicht brechen oder knicken.

Mittels der in Fig. 7 dargestellten Messvorrichtung kann gemessen werden, ob ein Applikationsstab eine solche Bruchfestigkeit aufweist.

Die Bruchfestigkeit kann an einem Applikationsstab mit daran befestigtem Wundreinigungskopf gemessen werden.

Der Applikationsstab 20 wird wie oben beschrieben auf das Auflager 310 des Prüfgeräts 300 aufgelegt, und die Druckfinne 320 wird zum Ausüben einer Biegekraft auf den Biegeabschnitt 26 des Applikationsstabs in den Zwischenraum zwischen den beiden Auflageflächen 311, 312 mit einer Stützweite St zwischen 40 mm und 60 mm, insbesondere etwa 48 mm, hineinbewegt.

Wenn der Applikationsstab 20 bei einer Durchbiegung von s=20 mm noch nicht bricht oder knickt, widersteht er einer Biegedehnung von 16% oder mehr und erfüllt die erfindungsgemäß bevorzugten Eigenschaften im Hinblick auf seine Bruchfestigkeit.

Der Wundreinigungskopf 50 ist bei der erfindungsgemäßen Applikationshilfe 10 mit einer hohen Verbindungsfestigkeit von 70 N oder mehr an dem Applikationsstab 20 befestigt. Mit anderen Worten löst sich der Wundreinigungskopf selbst dann noch nicht von dem Applikationsstab 20, wenn der Wundreinigungskopf 50 mit einer Zugkraft von 70 N, 75 N oder mehr in Längsrichtung des Applikationsstabs 20 nach oben (siehe Fig. 1) gezogen wird. Bei einer praktischen Anwendung der Applikationshilfe 10 zur Reinigung einer Wunde wird dadurch ausgeschlossen, dass sich der Wundreinigungskopf 50 beim Herausziehen aus der Wunde von dem Applikationsstab 20 löst. Zur sicheren Verbindung des Wundreinigungskopfs 50 an dem Applikationsstab 20 hat sich dabei eine Verbindungsfestigkeit von 70 N als besonders hilfreich erwiesen. In der Praxis kann aber auch eine Verbindungsfestigkeit in einem Bereich von 50 N oder mehr ausreichend sein.

Die Verbindungsfestigkeit zwischen dem Applikationsstab 20 und dem Wundreinigungskopf 50 kann mittels eines Zugprüfgeräts gemessen werden, das eine Halterung zum Fixieren des Applikationsstabs und eine Abzugsvorrichtung zum Ziehen an dem Wundreinigungskopf aufweist, wobei die Halterung und die Abzugsvorrichtung mit einer vorgegebenen Zugkraft auseinander gezogen werden können. Die zum Ablösen des Wundreinigungskopfs von dem Applikationsstab erforderliche Zugkraft kann auf diese Weise bestimmt werden.

Der Wundreinigungskopf 50 kann mittels einer oder mehrerer Befestigungsnahten an dem Applikationsstab 20 befestigt sein. Bspw. ist der Wundreinigungskopf am unteren Ende beidseitig und am oberen Ende vernäht.

Zusätzlich kann der Wundreinigungskopf 50 durch eine Öffnung 29 des Applikationsstabs 20 hindurch vernäht sein. Bspw. ist eine oberhalb der Öffnung 29 angeordnete Lage des Wundreinigungskopfs 50 mit einer unterhalb der Öffnung angeordneten Lage des Wundreinigungskopfs 50 vernäht, um die Verbindung zwischen dem Applikationsstab 20 und dem Wundreinigungskopf abzusichern.

Zumindest ein Nähfaden kann dabei eine Röntgenkontrastfähigkeit aufweisen und bspw. als mit Bariumsulfat beladener Kunststoffaden ausgebildet sein. Dieser RK-Faden (nicht dargestellt) umläuft bspw. den Applikationsstab 20 an dem unteren Ende des Wundreinigungskopfs in Fig. 1.

Der Wundreinigungskopf 50 kann ferner eine von dem Applikationsstab 20 durchlaufene Öffnung 51 an seinem in Fig. 1 unteren Ende ausbilden, wobei ein Herausziehen des Applikationsstabs 20 durch die Öffnung 51 durch Vorsprünge 27 oder Widerhaken verhindert wird, die von dem Applikationsstab 20 in einer Breitenrichtung nach außen vorstehen.

Zusätzlich kann der Wundreinigungskopf mittels Ultraschallverschweißen an dem Applikationsstab 20 angebracht sein.

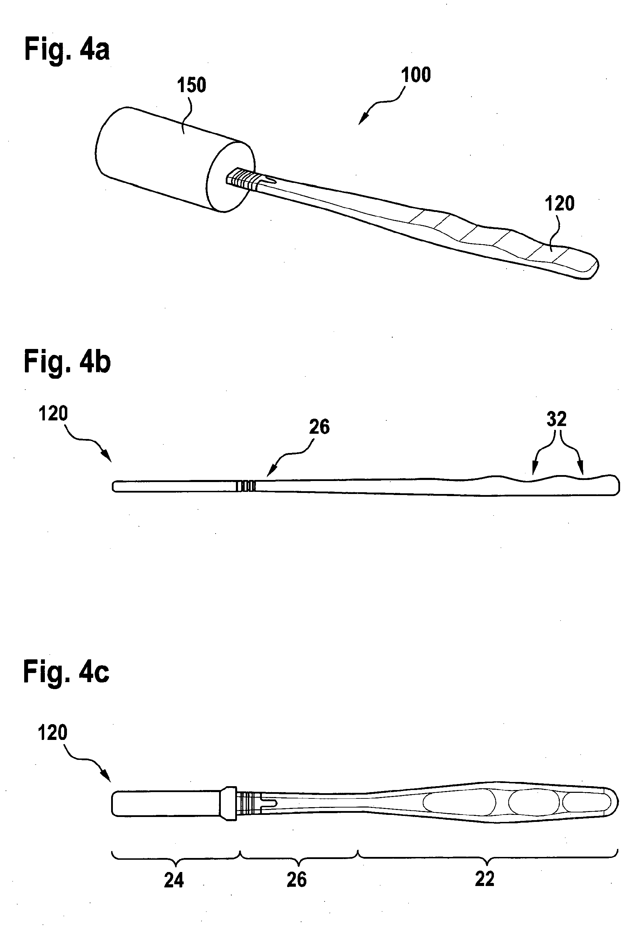

Fig. 4a zeigt eine weitere Ausführungsform einer erfindungsgemäßen Applikationshilfe 100, die einen Applikationsstab 120 und einen an dessen Befestigungsabschnitt angebrachten Wundreinigungskopf 150 aufweist. Fig. 4b zeigt den Applikationsstab 120 in einer ersten Seitenansicht, und Fig. 4c zeigt den Applikationsstab 120 in einer um 90° gedrehten Seitenansicht.

Es ist in den Figuren 4b und 4c deutlich erkennbar, dass der hintere Abschnitt des Applikationsstabs als Haltegriffabschnitt mit einer Profilierung 32 in Form von Wellungen ausgebildet ist. Im Hinblick auf eine besonders ergonomische Form mit guter Rutschfestigkeit verjüngt sich der Querschnitt des Haltegriffabschnitts in Richtung des Biegeabschnitts 26.

Zum Erhöhen der Biegsamkeit des Applikationsstabs 120 weist dieser in dem Biegeabschnitt 26 Einkerbungen bzw. Vertiefungen auf. Diese Einkerbungen sind in einem Bereich des Biegeabschnitts 26 angeordnet, der einen anderen Werkstoff aufweist, als die an den Biegeabschnitt 26 angrenzenden Bereiche. Dieser Werkstoff ist derart gewählt, dass er dem Biegeabschnitt 26 eine erhöhte Biegsamkeit verleiht. Dieser Werkstoff kann beispielsweise ein Elastomer aufweisen.

Der Applikationsstab 120 erstreckt sich im Wesentlichen linear und weist keine Biegung auf.

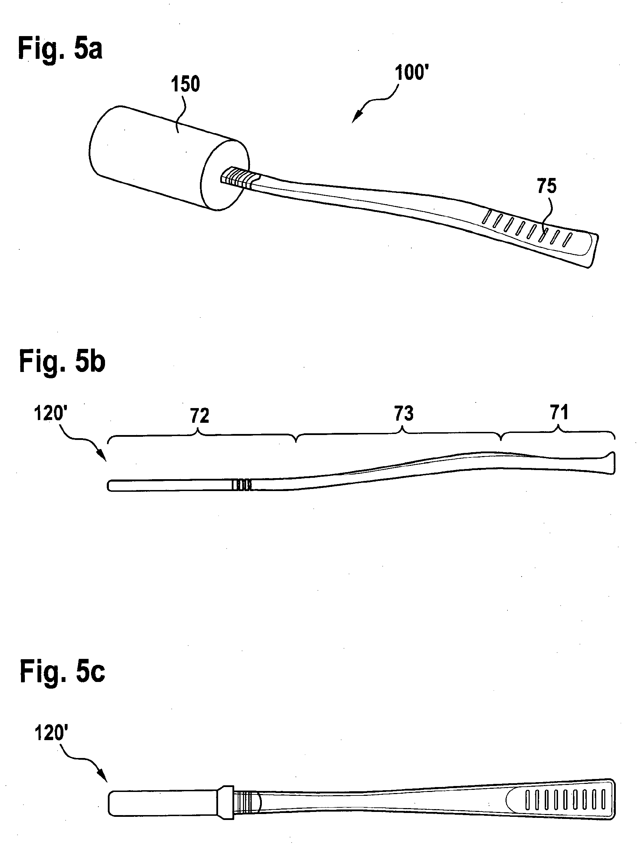

Fig. 5a zeigt eine weitere Ausführungsform einer erfindungsgemäßen Applikationshilfe 100', die einen Applikationsstab 120' und einen an dessen Befestigungsabschnitt angebrachten Wundreinigungskopf 150 aufweist. Fig. 5b zeigt den Applikationsstab 120' in einer ersten Seitenansicht, und Fig. 5c zeigt den Applikationsstab 120' in einer um 90° gedrehten Seitenansicht. Der Applikationsstab 120' entspricht im Wesentlichen den oben beschriebenen Applikationsstäben 20, 120, so dass auf die obigen Ausführungen verwiesen werden kann.

Im Unterschied zu dem Applikationsstab 120 aus Fig. 4b ist der Applikationsstab 120' zur Verbesserung der Handhabbarkeit abschnittsweise gekrümmt. In der in Fig. 5b gezeigten Seitenansicht ist erkennbar, dass der Längsverlauf des Applikationstabs an eine Kellenform angenähert ist, bei der ein hinterer Abschnitt 71 des Applikationsstabs im Wesentlichen in einer ersten Ebene und ein vorderer Abschnitt 72 des Applikationsstabs im Wesentlichen in einer bzgl. der ersten Ebene versetzten zweiten Ebene verläuft, wobei der mittlere Abschnitt 73 des Applikationsstabs von der ersten Ebene in die zweite Ebene gekrümmt verläuft.

Anstelle der als Wellung ausgebildeten Profilierung 32 weist der Applikationsstab 120' eine Riffelung 75 zum Erhöhen der Rutschfestigkeit auf.

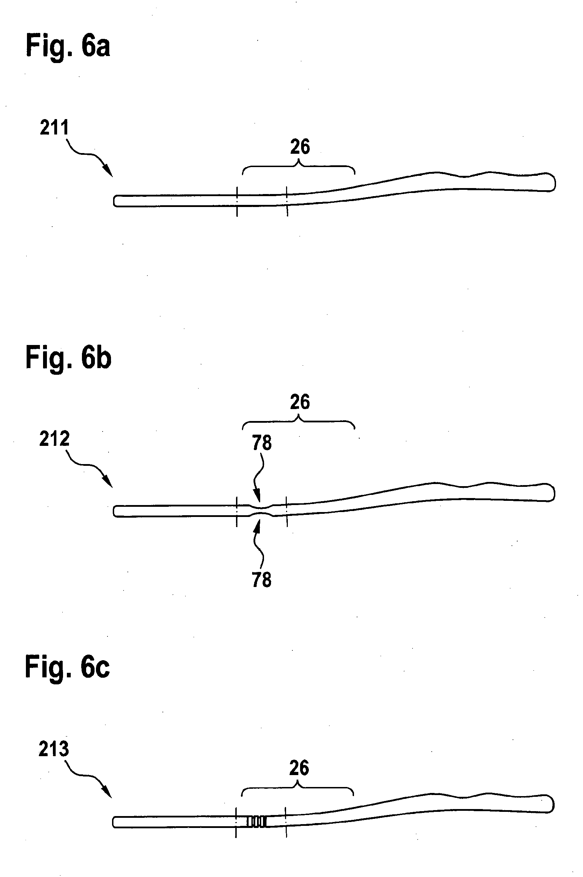

In den Figuren 6a bis 6c sind verschiedene Applikationsstäbe 211, 212, 213 erfindungsgemäßer Applikationshilfen jeweils in einer Seitenansicht dargestellt.

Während der in Fig. 6a dargestellte Applikationsstab 211 in seinem Biegeabschnitt 26 einen im Wesentlichen konstanten Stabquerschnitt hat, weist der in Fig. 6b dargestellte Applikationsstab eine Verjüngung in Form von zwei von entgegengesetzten Seiten des Applikationsstabs in den Biegeabschnitt 26 eingebrachten Kerben 78 auf, wobei die Verlaufsrichtung der Kerben senkrecht zu einer Hauptbiegerichtung des Applikationsstabs verläuft. Der in Fig. 6c dargestellte Applikationsstab weist mehrere umlaufende Einkerbungen 79 auf, die die Biegsamkeit des Applikationsstabs in beiden senkrecht zu der Längsrichtung des Applikationsstabs verlaufenden Querrichtungen erhöhen. Diese Einkerbungen 79 sind in einem Bereich des Biegeabschnitts 26 ausgebildet, der einen anderen Werkstoff aufweist als die an den Biegeabschnitt 26 angrenzenden Bereiche. Dieser Werkstoff ist derart beschaffen, dass er dem Biegeabschnitt 26 eine erhöhte Biegsamkeit verleiht. Dieser Werkstoff kann beisppielsweise ein Elastomer aufweisen.

Im Hinblick auf den Aufbau und die Merkmale des Wundreinigungskopfs 50 wird auf die Druckschrift WO 2010/085831 A1 verwiesen. Vorzugsweise weist der Wundreinigungskopf Polyesterfasern, vorzugsweise Polyesterfasern aus Polypropylen, zum effektiven Entfernen von Debris und Exsudat aus der Wunde sowie von Schuppen und Keratosen aus der Wundumgebungshaut auf. Ein weicher Faserverbund kann für eine schmerzarme Anwendung bei hohem Anwendungskomfort führen.

Der bruchsichere Griff, vorzugsweise aus Polypropylen, bietet einfachen Zugang zu tiefen Wunden und schwer zu erreichenden Wundlokalisationen.

Höchste Anwendungssicherheit kann durch präzise Befestigungsnähte, einen ergonomischen Griff und nachverfolgbare Röntgenkontrastfäden gewährleistet werden.

Vor der Reinigung einer Wunde kann der Wundreinigungskopf mit einer Wundspüllösung benässt werden.

Die erfindungsgemäße Applikationshilfe ist besonders gut für das Debridement von akuten und chronischen, oberflächlichen bis tiefen Wunden - auch chirurgisch invasiv - geeignet. Frühere Probleme in der Anwendung aufgrund einer zu hohen oder einer zu geringen Durchbiegung des Applikationsstabs können vermieden werden.

**Patentansprüche**

1. Applikationshilfe (10, 100, 100') für die Behandlung von Wunden, insbesondere für das Debridement und/oder die Reinigung von Wunden, mit einem biegbaren Applikationsstab (20, 120, 120', 211, 212, 213), bei dem ein hinterer Abschnitt des Applikationsstabs (20) als ein Haltegriffabschnitt (22) und/oder ein vorderer Abschnitt des Applikationsstab als ein Befestigungsabschnitt (24) zum Befestigen eines Wundreinigungskopfs (50, 150) ausgebildet ist, **gekennzeichnet durch** einen zwischen dem Haltegriffabschnitt (22) und dem Befestigungsabschnitt (24) bereitgestellten Biegeabschnitt (26) mit erhöhter Biegsamkeit, wobei sich der Applikationsstab (20) in einer 3-Punkt-Biegevorrichtung mit einer Stützweite zwischen 40 mm und 60 mm bei Einwirkung einer Biegekraft zwischen 21 N und 45 N, insbesondere von etwa 33 N, auf den Biegeabschnitt senkrecht zu der Längsrichtung des Applikationsstabs um etwa 5 bis 8 mm durchbiegt.

2. Applikationshilfe nach Anspruch 1, **dadurch gekennzeichnet, dass** der Applikationsstab (20) in dem Biegeabschnitt (26) einen gegenüber dem Haltegriffabschnitt (22) verkleinerten Stabquerschnitt und/oder eine sich quer zur Längsrichtung des Applikationsstabs erstreckende Kerbe oder Vertiefung zum Erhöhen der Biegsamkeit aufweist.

3. Applikationshilfe nach einem der Ansprüche 1 oder 2, **dadurch gekennzeichnet, dass** sich der Haltegriffabschnitt (22) über mehr als 1/3 und weniger als 2/3 der Länge (L) des Applikationsstabs (20) erstreckt und/oder sich der Befestigungsabschnitt (24) über mehr als 1/5 und weniger als 1/3 der Länge des Applikationsstabs (20) erstreckt, wobei die Länge des Befestigungsabschnitts (24) bevorzugt etwa 5 cm beträgt.

4.  Applikationshilfe nach einem der vorhergehenden Ansprüche, **dadurch gekennzeichnet, dass** sich eine Biegedehnung des Applikationsstabs (20) von 5% bei Einwirkung einer Biegekraft auf den Applikationsstab senkrecht zu dessen Längsrichtung von 60 N oder weniger, vorzugsweise 45 N oder weniger, besonders bevorzugt 39 N oder weniger und/oder 15 N oder mehr, vorzugsweise 21 N oder mehr, besonders bevorzugt 27 N oder mehr, insbesondere von etwa 33 N ergibt.

5.  Applikationshilfe nach einem der vorhergehenden Ansprüche, **dadurch gekennzeichnet, dass** der Applikationsstab (20) bruchfest ausgebildet ist, insbesondere dass die Bruchdehnung des Applikationsstabs 6% oder mehr, vorzugsweise 8% oder mehr und/oder 50% oder weniger, insbesondere etwa 16% beträgt.

6.  Applikationshilfe nach einem der vorhergehenden Ansprüche, **gekennzeichnet durch** einen an einem vorderen Abschnitt des Applikationsstabs befestigten Wundreinigungskopf (50), insbesondere in Form eines Wundreinigungspads oder Wundreinigungstuchs.

7.  Applikationshilfe nach Anspruch 6, **dadurch gekennzeichnet, dass** der Wundreinigungskopf (50) ein Faserpad, insbesondere ein Faserpad mit synthetischen Fasern aufweist, die vorzugsweise eine Faserstärke zwischen 0,5 und 20 dtex aufweisen.

8.  Applikationshilfe nach Anspruch 7, **dadurch gekennzeichnet, dass** das Faserpad zumindest eine Trägerschicht und an der Trägerschicht angeordnete und von der Trägerschicht abstehende Fäden aus synthetischen Fasern, insbesondere Kunststofffasern aufweist, die vorzugsweise ein Flor mit einer Florhöhe zwischen 3 und 30 mm bilden.

9.  Applikationshilfe nach Anspruch 8, **dadurch gekennzeichnet, dass** die Fäden schräg zu ihrer Längserstreckung verlaufende und vorzugsweise abgeschnittene Enden bzw. Endflächen aufweisen.

10. Applikationshilfe nach einem der Ansprüche 6 bis 9, **dadurch gekennzeichnet, dass** der Wundreinigungskopf (50) den Befestigungsabschnitt (24) des Applikationsstabs (20) vorzugsweise vollständig umläuft oder umhüllt.

11. Applikationshilfe nach einem der Ansprüche 6 bis 10, **gekennzeichnet durch** eine Verbindungsfestigkeit zwischen dem Wundreinigungskopf (50) und dem Applikationsstab (20) bei einem Abziehen des Wundreinigungskopfs von dem Applikationsstab in Längsrichtung des Applikationsstabs von 25 N oder mehr, vorzugsweise 50 N oder mehr, insbesondere 70 N oder mehr.

12. Applikationshilfe nach einem der Ansprüche 6 bis 11, **dadurch gekennzeichnet**, der Wundreinigungskopf eine von dem Applikationsstab (20) durchlaufene Durchgriffsöffnung (51) aufweist, wobei der Befestigungsabschnitt (24) des Applikationsstabs von dem Applikationsstab abstehende Vorsprünge (27) wie etwa Widerhaken aufweist, die ein Hindurchgleiten des Applikationsstabs durch die Durchgriffsöffnung (51) verhindern.

13. Applikationshilfe nach einem der Ansprüche 6 bis 12, **dadurch gekennzeichnet, dass** der Wundreinigungskopf (50) mittels einer oder mehrerer Befestigungsnahten an dem Applikationsstab (20) befestigt ist.

14. Applikationshilfe nach Anspruch 13, **dadurch gekennzeichnet, dass** ein dem Haltegriffabschnitt (22) des Applikationsstabs (20) zugewandter Rand (52) des Wundreinigungskopfs (50) einseitig oder beidseitig des Applikationsstabs eine Befestigungsnaht aufweist, und/oder dass ein vorderer Rand (54) des Wundreinigungskopfs zumindest eine Befestigungsnaht aufweist.

15. Applikationshilfe nach einem der Ansprüche 13 bis 14, **dadurch gekennzeichnet, dass** zumindest eine der Befestigungsnahten einen Röntgenkontrastfaden, insbesondere einen RK-Faden aufweist.

16. Applikationshilfe nach einem der Ansprüche 12 bis 15, **dadurch gekennzeichnet, dass** der Applikationsstab eine Öffnung (29) aufweist, durch die eine auf einer ersten Seite des Applikationsstabs angeordnete Lage des Wundreinigungskopfs (50) mit einer auf einer zweiten Seite des Applikationsstabs angeordneten Lage des Wundreinigungskopfs vernäht ist.

17. Applikationshilfe nach einem der Ansprüche 6 bis 16, **dadurch gekennzeichnet, dass** der Wundreinigungskopf mittels eines Haftmittels an dem Applikationsstab befestigt ist, insbesondere an den Applikationsstab geklebt ist.

18. Applikationshilfe nach einem der Ansprüche 6 bis 17, **dadurch gekennzeichnet, dass** der Wundreinigungskopf mittels Ultraschallschweißen an dem Applikationsstab befestigt ist.

19. Applikationshilfe nach einem der Ansprüche 6 bis 18, **dadurch gekennzeichnet, dass** der Wundreinigungskopf mittels eines vorzugsweise lösbaren Klicksystems an dem Applikationsstab befestigt ist.

20. Applikationshilfe nach einem der Ansprüche 6 bis 19, **dadurch gekennzeichnet, dass** der Wundreinigungskopf mittels eines Klettverschlusses an dem Applikationsstab befestigt ist.

21. Applikationshilfe nach einem der Ansprüche 6 bis 20, **dadurch gekennzeichnet, dass** der Wundreinigungskopf mittels eines Formverschlusses an dem Applikationsstab befestigt ist.

22. Applikationshilfe nach einem der Ansprüche 6 bis 21, **dadurch gekennzeichnet, dass** der Wundreinigungskopf mittels eines oder mehrerer Widerhaken an dem Applikationsstab befestigt ist.

23. Applikationshilfe nach einem der Ansprüche 6 bis 22, **dadurch gekennzeichnet, dass** die Länge (X) des Wundreinigungskopfs (50) in der Längsrichtung des Applikationsstabs 3 cm oder mehr und 10 cm oder weniger, insbesondere etwa 5 cm beträgt.

24. Applikationshilfe nach einem der Ansprüche 6 bis 23, **dadurch gekennzeichnet, dass** die Breite (B) des Wundreinigungskopfs (50) senkrecht zu der Längsrichtung des Applikationsstabs (20) 1 cm oder mehr und 5 cm oder weniger, insbesondere etwa 2 cm beträgt.

25. Applikationshilfe nach einem der vorhergehenden Ansprüche, **dadurch gekennzeichnet, dass** der Applikationsstab (20) eine Längenskala (31) zum Ablesen einer Wundtiefe aufweist.

26. Applikationshilfe nach einem der vorhergehenden Ansprüche, **dadurch gekennzeichnet, dass** ein Haltegriffabschnitt (22) des Applikationsstabs eine Profilierung (32), insbesondere eine Wellung, ein sich in Richtung auf das hintere Ende des Applikationsstabs (20) zumindest abschnittsweise erweiterndes Querschnittprofil und/oder eine ergonomische Form zum Erhöhen einer Rutschfestigkeit hat.

27. Applikationshilfe nach einem der vorhergehenden Ansprüche, **dadurch gekennzeichnet, dass** der Haltegriffabschnitt (22) des Applikationsstabs (20) zum Verbessern der Handhabbarkeit gebogen ist.

28. Applikationshilfe nach einem der vorhergehenden Ansprüche, **dadurch gekennzeichnet, dass** der Applikationsstab (20) aus einem Kunststoff, insbesondere aus Polypropylen gebildet ist.

29. Applikationshilfe nach einem der vorhergehenden Ansprüche, **dadurch gekennzeichnet, dass** die Länge (L) des Applikationsstabs 10 cm oder mehr und 20 cm oder weniger, insbesondere etwa 16 cm beträgt.

30. Applikationshilfe nach einem der Ansprüche 3 bis 29, **dadurch gekennzeichnet, dass** ein Bereich des Biegeabschnitts (26) einen Werkstoff geringerer Biegesteifigkeit aufweist als die an den Biegeabschnitt (26) angrenzenden Abschnitt (22, 24).

**Claims**

1. An application aid (10, 100, 100') for the treatment of wounds, in particular for the debridement and/or cleansing of wounds, comprising a bendable application wand (20, 120, 120', 211, 212, 213), whereby a rear section of the application wand (20) is designed as a grasping handle section (22) and/or a front section of the application wand is designed as a fastening section (24) for fastening a wound cleansing head (50, 150) thereto, **characterised by** a bending section (26) with increased flexibility provided between the grasping handle section (22) and the fastening section (24), wherein the application wand (20) in a 3-point bending device with a span between supports of between 40 mm and 60 mm deflects by approximately 5 mm to 8 mm on application of a bending force of between 21 N and 45 N, in particular of approximately 33 N, on the bending section perpendicular to the longitudinal direction of the application wand.

2. The application aid according to claim 1, **characterised in that** the application wand (20) in the bending section (26) has a reduced wand cross section compared to the grasping handle section (22) and/or a notch or recess to increase flexibility, said notch or recess extending transverse to the longitudinal direction of the application wand.

3. The application aid according to one of claims 1 or 2, **characterised in that** the grasping handle section (22) extends over more than 1/3 but less than 2/3 of the length (L) of the application wand (20) and/or the fastening section (24) extends over more than 1/5 but less than 1/3 of the length of the application wand (20), wherein the length of the fastening section (24) is preferably approximately 5 cm.

4. The application aid according to one of the preceding claims, **characterised in that** a flexural elongation of the application wand (20) of 5% is produced on exertion on the application rod perpendicular to its longitudinal of a bending force of 60 N or less, preferably 45 N or less, particularly preferably 39 N or less and/or 15 N or more, preferably 21 N or more, particularly preferably 27 N or more, in particular of approximately 33 N.

5. The application aid according to one of the preceding claims, **characterised in that** the application wand (20) is designed to be break-proof, particularly **in that** the elongation at break of the application wand is 6% or more, preferably 8% or more and/or 50% or less, in particular approximately 16%.

6. The application aid according to one of the preceding claims, **characterised by** a wound cleansing head (50) fastened to a front section of the application wand, in particular in the form of a wound cleansing pad or fibre wound cleansing wipe.

7. The application aid according to claim 6, **characterised in that** the wound cleansing head (50) has a fibre pad, in particular a fibre pad with synthetic fibres preferably with a fibre strength of between 0.5 dtex and 20 dtex.

8. The application aid according to claim 7, **characterised in that** the fibre pad comprises at least one support layer and threads made from synthetic fibres, in particular plastic fibres, arranged on the support layer, the threads projecting from the support layer to preferably form a pile with a pile height of between 3 mm and 30 mm.

9. The application aid according to claim 8, **characterised in that** the threads have ends or end faces that run obliquely to their longitudinal extent and are preferably cut off.

10. The application aid according to one of claims 6 to 9, **characterised in that** the wound cleansing head (50) preferably completely encircles or envelopes the fastening section (24) of the application wand (20).

11. The application aid according to one of claims 6 to 10, **characterised by** a joint strength between the wound cleansing head (50) and the application wand (20) when the wound cleansing head is removed from the application wand in the longitudinal direction of the application wand of 25 N or more, preferably 50 N or more, in particular 70 N or more.

12. The application aid according to one of claims 6 to 11, **characterised in that** the wound cleansing head has a reach-through opening (51) through which the application wand (20) is inserted, wherein the fastening section (24) of the application wand comprises projections (27) protruding out from the application wand, such as barbs, which help keep the inserted application wand from sliding back through the reach through opening (51).

13. The application aid according to one of claims 6 to 12, **characterised in that** the wound cleansing head (50) is fastened to the application wand (20) with one or more fastening seams.

14. The application aid according to claim 13, **characterised in that** an edge (52) of the wound cleansing head (50) facing the grasping handle section (22) of the application wand (20) has a fastening seam on one or both sides of the application wand, and/or **in that** a front edge (54) of the wound cleansing head has at least one fastening seam.

15. The application aid according to one of claims 13 and 14, **characterised in that** at least one of the fastening seams comprises an X-ray contrast thread.

16. The application aid according to one of claims 12 to 15, **characterised in that** the application wand has an opening (29) through which a layer of the wound cleansing head (50) arranged on a first side of the application wand is sewn to a layer of the wound cleansing head arranged on a second side of the application wand.

17. The application aid according to one of claims 6 to 16, **characterised in that** the wound cleansing head is fastened to the application wand with an adhesive, in particular is bonded to the application wand.

18. The application aid according to one of claims 6 to 17, **characterised in that** the wound cleansing head is fastened to the application wand by means of ultrasonic welding.

19. The application aid according to one of claims 6 to 18, **characterised in that** the wound cleansing head is fastened to the application wand by means of a preferably releasable click system.

20. The application aid according to one of claims 6 to 19, **characterised in that** the wound cleansing head is fastened to the application wand by means of a hook and loop fastener.

21. The application aid according to one of claims 6 to 20, **characterised in that** the wound cleansing head is fastened to the application wand in a form-locking manner.

22. The application aid according to one of claims 6 to 21, **characterised in that** the wound cleansing head is fastened to the application wand by means of one or more barbs.

23. The application aid according to one of claims 6 to 22, **characterised in that** the length (X) of the wound cleansing head (50) in the longitudinal direction of the application wand is 3 cm or more and 10 cm or less, in particular approximately 5 cm.

24. The application aid according to one of claims 6 to 23, **characterised in that** the width (B) of the wound cleansing head (50) perpendicular to the longitudinal direction of the application wand (20) is 1 cm or more and 5 cm or less, in particular approximately 2 cm.

25. The application aid according to one of the preceding claims, **characterised in that** the application wand (20) comprises a length scale (31) for determining the depth of a wound.

26. The application aid according to one of the preceding claims, **characterised in that** a grasping handle section (22) of the application wand comprises a profile (32), in particular a corrugated profile, a cross-sectional profile that increases at least in portions towards the rear end of the application wand (20) and/or an ergonomic shape to increase slip resistance.

27. The application aid according to one of the preceding claims, **characterised in that** the grasping handle section (22) of the application wand (20) is curved to improve handling.

28. The application aid according to one of the preceding claims, **characterised in that** the application wand (20) is made from a plastic, in particular from polypropylene.

29. The application aid according to one of the preceding claims, **characterised in that** the length (L) of the application wand is 10 cm or more and 20 cm or less, in particular approximately 16 cm.

30. The application aid according to one of claims 3 to 29, **characterised in that** a portion of the bending section (26) comprises a material of lower flexural rigidity than the sections (22, 24) adjoining the bending section (26).

**Revendications**

1. Dispositif applicateur (10, 100, 100') destiné au traitement de plaies, notamment au débridement et/ou au nettoyage de plaies, comportant une tige d'application flexible (20, 120, 120', 211, 212, 213), une partie postérieure de ladite tige d'application (20) consistant en une partie formant manche (22) et/ou une partie antérieure de la tige d'application consistant en une partie de fixation (24) destinée à la fixation d'une tête de nettoyage de plaie (50, 150), le dispositif étant **caractérisé par** une partie flexible (26) dotée d'une flexibilité accrue, située entre la partie formant manche (22) et la partie de fixation (24), ladite tige d'application (20) fléchissant d'environ 5 à 8 mm dans le cadre d'un dispositif de flexion à 3 points mettant en œuvre un écart entre les supports compris entre 40 mm et 60 mm, avec une force de flexion comprise entre 21 N et 45 N, notamment d'environ 33 N, appliquée sur la partie flexible perpendiculairement à la direction longitudinale de la tige d'application.

2. Dispositif applicateur selon la revendication 1, **caractérisé en ce que** la tige d'application (20), dans la partie flexible (26), présente une section transversale réduite par rapport à celle de la partie formant manche (22) et/ou une rainure ou un renfoncement dirigé transversalement à la direction longitudinale de la tige d'application et visant à accroître sa flexibilité.

3. Dispositif applicateur selon l'une des revendications 1 et 2, **caractérisé en ce que** la partie formant manche (22) s'étend sur plus d'un tiers et moins des deux tiers de la longueur (L) de la tige d'application (20) et/ou la partie de fixation (24) s'étend sur plus d'un cinquième et moins du tiers de la longueur de la tige d'application (20), la longueur de ladite partie de fixation (24) faisant de préférence environ 5 cm.

4. Dispositif applicateur selon l'une des revendications précédentes, **caractérisé en ce qu'**il se produit un allongement en flexion de la tige d'application (20) de 5 % sous l'effet d'une force de flexion, agissant sur la tige d'application perpendiculairement à sa direction longitudinale, de 60 N ou moins, de préférence de 45 N ou moins, plus préférablement de 39 N ou moins et/ou de 15 N ou plus, de préférence de 21 N ou plus, plus préférablement de 27 N ou plus, notamment d'environ 33 N.

5. Dispositif applicateur selon l'une des revendications précédentes, **caractérisé en ce que** la tige d'application (20) est conçue incassable, notamment **en ce que** l'allongement à la rupture de la tige d'application fait 6 % ou plus, de préférence 8 % ou plus et/ou 50 % ou moins, en étant notamment d'environ 16 %.

6. Dispositif applicateur selon l'une des revendications précédentes, **caractérisé par** une tête de nettoyage de plaie (50) fixée à une partie antérieure de la tige d'application, notamment sous la forme d'un tampon ou d'une lingette de nettoyage de plaie.

7. Dispositif applicateur selon la revendication 6, **ca-**

**ractérisé en ce que** la tête de nettoyage de plaie (50) présente un tampon fibreux, notamment un tampon fibreux à fibres synthétiques présentant de préférence une épaisseur comprise entre 0,5 et 20 dtex.

8. Dispositif applicateur selon la revendication 7, **caractérisé en ce que** le tampon fibreux présente au moins une couche support ainsi que des fils de fibres synthétiques, notamment de plastique, disposés sur la couche support et faisant saillie de ladite couche support en formant de préférence un velours d'une hauteur de fibre comprise entre 3 et 30 mm.

9. Dispositif applicateur selon la revendication 8, **caractérisé en ce que** les fils présentent des extrémités ou surfaces terminales obliques par rapport à leur étendue longitudinale, lesquelles sont de préférence coupées.

10. Dispositif applicateur selon l'une des revendications 6 à 9 **caractérisé en ce que** la tête de nettoyage de plaie (50) entoure ou enveloppe la partie de fixation (24) de la tige d'application (20), de préférence totalement.

11. Dispositif applicateur selon l'une des revendications 6 à 10, **caractérisé par** une solidité d'assemblage entre la tête de nettoyage de plaie (50) et la tige d'application (20), lors du retrait de la tête de nettoyage de plaie hors de la tige d'application dans une direction longitudinale de la tige d'application, qui est de 25 N ou plus, de préférence de 50 N ou plus, notamment de 70 N ou plus.

12. Dispositif applicateur selon l'une des revendications 6 à 11, **caractérisé en ce que** la tête de nettoyage de plaie présente une ouverture de passage (51) traversée par la tige d'application (20), ladite partie de fixation (24) de la tige d'application présentant des épaulements (27) faisant saillie à partir de la tige d'application, tels que des barbillons, qui empêchent un coulissement de la tige d'application par l'ouverture de passage (51).

13. Dispositif applicateur selon l'une des revendications 6 à 12, **caractérisé en ce que** la tête de nettoyage de plaie (50) est fixée à la tige d'application (20) au moyen d'une ou plusieurs coutures de fixation.

14. Dispositif applicateur selon la revendication 13, **caractérisé en ce qu'**un bord (52) de la tête de nettoyage de plaie (50) tournée vers la partie formant manche (22) de la tige d'application (20) présente une couture de fixation d'un côté de la tige d'application ou des deux, et/ou **en ce qu'**un bord avant (54) de la tête de nettoyage de plaie présente au moins une couture de fixation.

15. Dispositif applicateur selon l'une des revendications 13 et 14, **caractérisé en ce qu'**au moins une des coutures de fixation présente un fil de contraste aux rayons X.

16. Dispositif applicateur selon l'une des revendications 12 à 15, **caractérisé en ce que** la tige d'application présente une ouverture (29) à travers laquelle une couche de la tête de nettoyage de plaie (50) située sur un premier côté de la tige d'application est cousue à une couche de la tête de nettoyage de plaie située sur un deuxième côté de la tige d'application.

17. Dispositif applicateur selon l'une des revendications 6 à 16, **caractérisé en ce que** la tête de nettoyage de plaie est fixée à la tige d'application au moyen d'un adhésif et est notamment collée à ladite tige d'application.

18. Dispositif applicateur selon l'une des revendications 6 à 17, **caractérisé en ce que** la tête de nettoyage de plaie est fixée à la tige d'application par soudage aux ultrasons.

19. Dispositif applicateur selon l'une des revendications 6 à 18, **caractérisé en ce que** la tête de nettoyage de plaie est fixée à la tige d'application au moyen d'un système d'encliquetage qui est de préférence déblocable.

20. Dispositif applicateur selon l'une des revendications 6 à 19, **caractérisé en ce que** la tête de nettoyage de plaie est fixée à la tige d'application au moyen d'un système auto-agrippant.

21. Dispositif applicateur selon l'une des revendications 6 à 20, **caractérisé en ce que** la tête de nettoyage de plaie est fixée à la tige d'application par complémentarité de forme.

22. Dispositif applicateur selon l'une des revendications 6 à 21, **caractérisé en ce que** la tête de nettoyage de plaie est fixée à la tige d'application au moyen d'un ou plusieurs barbillons.

23. Dispositif applicateur selon l'une des revendications 6 à 22, **caractérisé en ce que** la longueur (X) de la tête de nettoyage de plaie (50) dans la direction longitudinale de la tige d'application fait 3 cm ou plus et 10 cm ou moins, en étant notamment d'environ 5 cm.

24. Dispositif applicateur selon l'une des revendications 6 à 23, **caractérisé en ce que** la largeur (B) de la tête de nettoyage de plaie (50) perpendiculairement à la direction longitudinale de la tige d'application (20) fait 1 cm ou plus et 5 cm ou moins, en étant notamment d'environ 2 cm.

**25.** Dispositif applicateur selon l'une des revendications précédentes, **caractérisé en ce que** la tige d'application (20) présente une échelle graduée (31) permettant de relever la profondeur d'une plaie.

**26.** Dispositif applicateur selon l'une des revendications précédentes, **caractérisé en ce qu'**une partie formant manche (22) de la tige d'application possède un profilage (32), notamment une ondulation, un profil en section transversale augmentant de volume, au moins par endroits, en direction de l'extrémité postérieure de la tige d'application (20) et/ou une forme ergonomique résistant davantage au dérapage.

**27.** Dispositif applicateur selon l'une des revendications précédentes, **caractérisé en ce que** la partie formant manche (22) de la tige d'application (20) est incurvée pour améliorer la maniabilité.

**28.** Dispositif applicateur selon l'une des revendications précédentes, **caractérisé en ce que** la tige d'application (20) est réalisée en plastique, notamment en polypropylène.

**29.** Dispositif applicateur selon l'une des revendications précédentes, **caractérisé en ce que** la longueur (L) de la tige d'application fait 10 cm ou plus et 20 cm ou moins, en étant notamment d'environ 16 cm.

**30.** Dispositif applicateur selon l'une des revendications 3 à 29, **caractérisé en ce qu'**une zone de la partie flexible (26) présente un matériau de moindre rigidité flexionnelle que celle des parties (22, 24) adjacentes à la partie flexible (26).

# Fig. 1

**Fig. 2**

# Fig. 3

## Fig. 4a

150

100

120

## Fig. 4b

120

26

32

## Fig. 4c

120

24

26

22

Fig. 5a

150

100'

75

Fig. 5b

120'

72

73

71

Fig. 5c

120'

# Fig. 6a

211

26

# Fig. 6b

26

212

78

78

# Fig. 6c

213

26

# Fig. 7

**22**  **26**  **320**  **24**  **330**

**20**

**312**

**310**

**311**

**300**

**320**

**St**

**s**

**EP 3 448 340 B1**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2010085831 A1